# EUROPEAN PATENT APPLICATION

(11) **EP 4 810 475 A1**
(43) Date of publication of application: **23.09.2026**
(21) Application number: 24891021.8
(22) Date of filing: 22.07.2024
(51) Int. Cl.: C07C 47/198, C07C 45/29, C11B 9/00

(54) **NOVEL ALDEHYDE COMPOUND**

(30) Priority: 15.11.2023 JP 2023194596
(71) Applicant: Kao Corporation, Tokyo 103-8210 (JP)
(72) Inventor: KOYAMA, Shiori, Tokyo 103-8210 (JP); DEGUCHI, Jun, Tokyo 103-8210 (JP); ARAI, Tsubasa, Tokyo 103-8210 (JP); OHNO, Satoshi, Tokyo 103-8210 (JP)
(74) Representative: Hoffmann Eitle
(86) International application number: PCT/JP2024/026117
(87) International publication number: WO 2025/104977

(57) **Abstract**

Provided are a novel compound having a woody odor that exhibits a minty and camphor-like character with slight earthy and floral-muguet facets and possesses nectar-like sweetness, and a fragrance composition. The compound is represented by a formula (I). [In the formula (I), R is an alkyl group having 1 or more and 5 or less carbon atoms.]

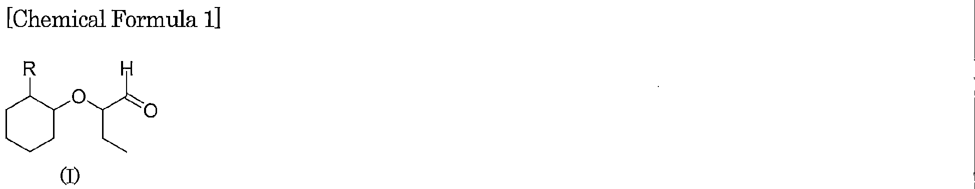

## Description

### Technical Field

The present invention relates to a compound represented by a general formula (I) and a fragrance composition containing the compound.

### Background Art

Aldehyde compounds are known to exhibit useful activities in pharmaceuticals, fragrances, and pesticides.

JP S61-268643A (Patent Document 1) discloses cis-(or trans-) 1-[1' (or 2' or 3')-formylpropoxy]-4-tert-butylcyclohexane and a fragrance composition containing the same. Patent Document 1 states that this fragrance composition has a green, aldehydic, citrus-like odor. 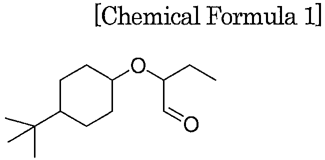 cis-(or trans-)1-[1'-formylpropoxy]-4-tert-butylcyclohexane

JP H5-339188A (Patent Document 2) discloses 2-(2-t-butylcyclohexyloxy)-1-butanol and a fragrance composition containing the same. Patent Document 2 states that this fragrance composition has a woody and amber-like odor with excellent odor persistence. 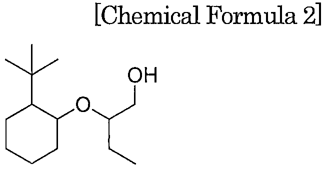 2-(2-t-butylcyclohexyloxy)-1-butanol

### Disclosure of Invention

The present invention provides a compound represented by a formula (I):

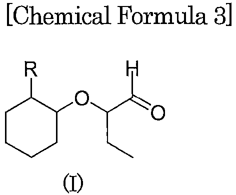

[In the formula (I),
R is an alkyl group having 1 or more and 5 or less carbon atoms.]

### Description of the Invention

An object of the present invention is to provide a novel compound having a woody odor and a fragrance composition containing the same. This woody odor exhibits a minty and camphor-like character with slight earthy and floral-muguet facets. This woody odor further possesses nectar-like sweetness.

The inventors of the present invention have found a novel aldehyde compound having a woody odor and thus devised the present invention. This woody odor exhibits a minty and camphor-like character with slight earthy and floral-muguet facets. This woody odor further possesses nectar-like sweetness.

The present invention provides a compound represented by a formula (I).

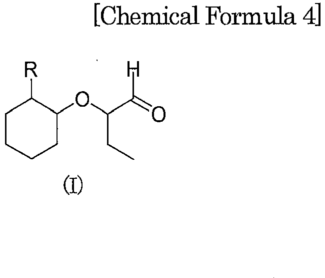

[In the formula (I),
R is an alkyl group having 1 or more and 5 or less carbon atoms.]

The present invention also provides a fragrance composition containing the compound represented by the formula (I).

The present invention further provides a method of using the compound represented by the formula (I) as a fragrance-imparting component.

The compound represented by the formula (I) of the present invention has a woody odor. This woody odor exhibits a minty and camphor-like character with slight earthy and floral-muguet facets. This woody odor further possesses nectar-like sweetness.

As described above, the present invention provides a compound represented by the formula (I).

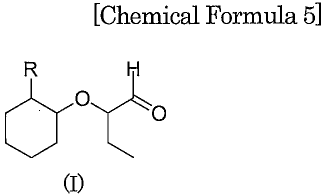

[In the formula (I),
R is an alkyl group having 1 or more and 5 or less carbon atoms.]

In the present specification, examples of the alkyl group having 1 or more and 5 or less carbon atoms include straight-chain or branched alkyl groups such as methyl, ethyl, n-propyl, i-propyl, n-butyl, i-butyl, sec-butyl, t-butyl, n-pentyl, i-pentyl, sec-pentyl, t-pentyl, and 2-methylbutyl groups. The alkyl group having 1 or more and 5 or less carbon atoms is preferably an alkyl group having 1 or more and 4 or less carbon atoms, more preferably an alkyl group having 3 or more and 4 or less carbon atoms, and still more preferably a t-butyl group.

In the formula (I), from the viewpoint of imparting a woody odor that exhibits a minty and camphor-like character with slight earthy and floral-muguet facets and possesses nectar-like sweetness,
R is preferably an alkyl group having 1 or more and 4 or less carbon atoms, more preferably an alkyl group having 3 or more and 4 or less carbon atoms, and still more preferably a t-butyl group.

The compound represented by the formula (I) can be produced using the following method, for example.

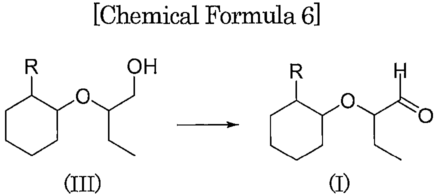

In the formulae (I) and (III),
R is an alkyl group having 1 or more and 5 or less carbon atoms.

The production method of the present invention includes a step of oxidizing a compound of the formula (III) to thereby obtain a compound of the formula (I). In this step, the compound of the formula (III) is subjected to, for example, oxidation using an oxidizing agent (such as, for example, Dess-Martine periodinane), Swern oxidation, PCC oxidation, TPAP (Ley-Griffith) oxidation, TEMPO oxidation, or oxidation using MnO₂ to obtain a compound of the formula (I). The thus obtained compound of the formula (I) can be separated and purified by distillation or column chromatography.

The present invention also provides a fragrance composition containing the compound represented by the formula (I).

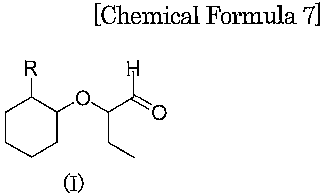

[In the formula (I),
R is an alkyl group having 1 or more and 5 or less carbon atoms.]

The fragrance composition containing the compound represented by the formula (I) can impart a woody odor that exhibits a minty and camphor-like character with slight earthy and floral-muguet facets and possesses nectar-like sweetness. Thus, it harmonizes well with various other fragrances and can bring about a characteristic effect on odors when used in fragrance blending. Specifically, the fragrance composition can impart naturalness to alcohol-based fragrance compounds. In other words, the compound represented by the formula (I) can impart to alcohol-based fragrance compounds that are not naturally derived fragrance compositions, such as extracts, a complexity comparable to that of natural fragrance compositions.

In the fragrance composition containing the compound represented by the formula (I), from the viewpoint of imparting a woody odor that exhibits a minty and camphor-like character with slight earthy and floral-muguet facets and possesses nectar-like sweetness (hereinafter also referred to simply as the "viewpoint of imparting a woody odor having a minty and camphor-like character and nectar-like sweetness"), R in the formula (I) is preferably an alkyl group having 1 or more and 4 or less carbon atoms, more preferably an alkyl group having 3 or more and 4 or less carbon atoms, and still more preferably a t-butyl group.

The content of the compound of the formula (I) in the fragrance composition is preferably 0.05 mass% or more, more preferably 0.5 mass% or more, and still more preferably 1 mass% or more; and is preferably 30 mass% or less, more preferably 20 mass% or less, and still more preferably 10 mass% or less, from the viewpoint of imparting a woody odor having a minty and camphor-like character and nectar-like sweetness. From the foregoing viewpoint, the content is preferably 0.05 mass% or more and 30 mass% or less, more preferably 0.5 mass% or more and 20 mass% or less, and still more preferably 1 mass% or more and 10 mass% or less.

Preferably, the fragrance composition further contains an alcohol having 8 or more and 14 or less carbon atoms. From the viewpoint of enabling the fragrance composition, by further containing an alcohol having 8 or more and 14 or less carbon atoms, to exhibit enhanced naturalness and a complexity comparable to that of a natural fragrance composition, thereby rendering the odor of a mixture of the fragrance composition further containing the alcohol having 8 or more and 14 or less carbon atoms and a fragrance blend closer to that of a natural fragrance, the alcohol having 8 or more and 14 or less carbon atoms is preferably a monohydric alcohol (mono-ol), and alcohol-based fragrance compounds are preferred. Alcohol-based fragrance compounds refer to compounds that are used for imparting fragrance and that contain a hydroxyl group.

Examples of the alcohol having 8 or more and 14 or less carbon atoms include Bacdanol (2-ethyl-4-(2,2,3-trimethyl-3-cyclopenten-1-yl)-2-buten-1-ol), cinnamic alcohol, citronellol, geraniol, phenethyl alcohol, dihydromyrcenol, Sandalmysore Core (2-methyl-4-(2,2,3-trimethyl-3-cyclopenten-1-yl)-2-buten-1-ol), Amber Core (1-(2-t-butyleyclohexyloxy)-2-butanol), linalool, myrcenol, nerol, tetrahydrolinalool, tetrahydrogeraniol, hydroxycitronellol, terpineol, isopulegol, nopol, thymol, eugenol, and 3-methyl-5-phenylpentanol. From the viewpoint of enabling the fragrance composition, by further containing the alcohol having 8 or more and 14 or less carbon atoms, to exhibit enhanced naturalness and a complexity comparable to that of a natural fragrance composition, thereby rendering the odor of a mixture of the fragrance composition further containing the alcohol having 8 or more and 14 or less carbon atoms and a fragrance blend closer to that of a natural fragrance, it is preferable to employ one or more selected from the group consisting of Bacdanol, Sandalmysore Core, and Amber Core.

[Chemical Formula 8]

| | | | |
|---|---|---|---|
| 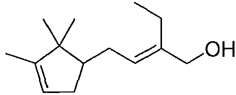 Bacdanol | 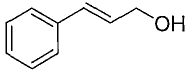 cinnamic alcohol | 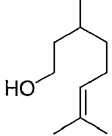 citronellol | 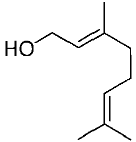 geraniol |
| 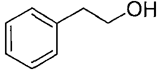 phenethyl alcohol | 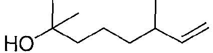 dihydromyrcenol | 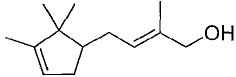 Sandalmysore Core | 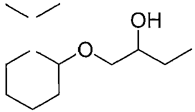 Amber Core |

The mass ratio of the compound represented by the formula (I) to the alcohol having 8 or more and 14 or less carbon atoms [compound represented by formula (I)/alcohol having 8 or more and 14 or less carbon atoms] is preferably 0.001 or more, more preferably 0.01 or more, still more preferably 0.05 or more, and yet still more preferably 0.07 or more, from the viewpoint of enabling the fragrance composition, by further containing the alcohol having 8 or more and 14 or less carbon atoms, to exhibit enhanced naturalness and a complexity comparable to that of a natural fragrance composition and the viewpoint of consequently rendering the odor of a mixture of the fragrance composition further containing the alcohol having 8 or more and 14 or less carbon atoms and a fragrance blend closer to that of a natural fragrance. The mass ratio is preferably 20 or less, more preferably 10 or less, still more preferably 5 or less, and yet still more preferably 1 or less, from the viewpoint of imparting the inherent odor characteristics of the alcohol having 8 or more and 14 or less carbon atoms. From these viewpoints, the mass ratio is preferably 0.001 or more and 20 or less, more preferably 0.01 or more and 10 or less, still more preferably 0.05 or more and 5 or less, and yet still more preferably 0.07 or more and 1 or less.

In addition, the content of the alcohol having 8 or more and 14 or less carbon atoms in the fragrance composition containing the compound represented by the formula (I) is preferably 1 mass% or more, more preferably 5 mass% or more, and still more preferably 10 mass% or more, from the viewpoint of enhancing naturalness and imparting a complexity comparable to that of a natural fragrance composition. From the same viewpoint, the content is preferably 90 mass% or less, more preferably 70 mass% or less, and still more preferably 50 mass% or less; and is preferably 1 mass% or more and 90 mass% or less, more preferably 5 mass% or more and 70 mass% or less, and still more preferably 10 mass% or more and 50 mass% or less.

Note that when multiple alcohols having 8 or more and 14 or less carbon atoms are present, the total mass of these alcohols is regarded as the "mass of the alcohol having 8 or more and 14 or less carbon atoms".

Preferably, the alcohol having 8 or more and 14 or less carbon atoms includes a compound represented by a formula (II).

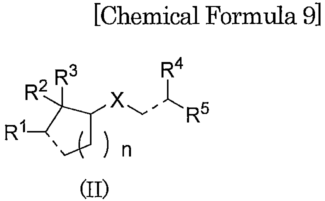

In the formula (II),
R¹ and R² are each independently a hydrogen atom or a methyl group,
R³ is a methyl group, ethyl group, n-propyl group, isopropyl group, or t-butyl group,
R⁴ is a methyl group or ethyl group,
R⁵ is a hydroxyl group or hydroxymethyl group,
X is -CH₂- or -O-,
n is 1 or 2, and
each dashed line independently represents a single or double bond.

From the viewpoint of enabling the fragrance composition, by further containing the alcohol having 8 or more and 14 or less carbon atoms, to exhibit enhanced naturalness and a complexity comparable to that of a natural fragrance composition, thereby rendering the odor of a mixture of the fragrance composition further containing the alcohol having 8 or more and 14 or less carbon atoms and a fragrance blend closer to that of a natural fragrance, it is preferable that the alcohol having 8 or more and 14 or less carbon atoms or the compound represented by the formula (II) is one or more selected from Bacdanol (2-ethyl-4-(2,2,3-trimethyl-3-cyclopenten-1-yl)-2-buten-1-ol), Sandalmysore Core (2-methyl-4-(2,2,3-trimethyl-3-cyclopenten-1-yl)-2-buten-1-ol), and Amber Core (1-(2-t-butylcyclohexyloxy)-2-butanol).

It is preferable that the fragrance composition of the present invention containing the compound represented by the formula (I) and the alcohol having 8 or more and 14 or less carbon atoms further contains at least one component that is other than the compound represented by the formula (I) and the alcohol having 8 or more and 14 or less carbon atoms and is selected from alcohols, phenols, esters, carbonates, aldehydes, ketones, acetals, ethers, lactones, nitriles, and amines.

In the present specification, each such fragrance "class" refers to either a single compound or a mixture of two or more compounds.

Examples of the alcohols include aliphatic alcohols, terpene alcohols, aromatic alcohols, and other alcohols. Of these, aliphatic alcohols are preferable.

Examples of the terpene alcohols include linalool, ethyl linalool, hydroxycitronellol, nerol, terpineol, α-terpineol, dihydromyrcenol, farnesol, nerolidol, cedrol, menthol, and borneol.

Examples of the aromatic alcohols include phenylethyl alcohol, benzyl alcohol, dimethyl benzyl carbinol, phenylethyl dimethyl carbinol, and phenyl hexanol.

Examples of the aliphatic alcohols include cis-3-hexenol, 1-(2,2,6-trimethylcyclohexyl)-3-hexanol, Magnol (trade name, manufactured by Kao Corporation; a mixture containing ethyl norbornyl cyclohexanol as a main component), Undecavertol (trade name, manufactured by Givaudan; 4-methyl-3-decen-5-ol), isobornyl cyclohexanol, and Terpirosa (trade name, manufactured by Kao Corporation; 3,6-dimethyl-2-heptanol).

Examples of other alcohols include glycols such as dipropylene glycol, Florosa (trade name, manufactured by Givaudan; chemical name: 4-methyl-2-(2-methylpropyl)tetrahydro-2H-4-pyranol), maltol, and ethyl maltol.

Examples of the hydrocarbons include limonene, α-pinene, β-pinene, terpinene, cedrene, longifolene, and valencene.

Examples of the phenols include guaiacol, eugenol, dihydroeugenol, isoeugenol, thymol, para-cresol, vanillin, and ethyl vanillin.

Examples of the esters include aliphatic carboxylic acid esters, aromatic carboxylic acid esters, and other carboxylic acid esters.

Examples of aliphatic carboxylic acids that form the aliphatic carboxylic acid esters include straight-chain and branched carboxylic acids having 1 to 18 carbon atoms. Of these, carboxylic acids having 1 to 6 carbon atoms, such as formic acid, acetic acid, and propionic acid, are preferable, and acetic acid is more preferable. Examples of aromatic carboxylic acids that form the aromatic carboxylic acid esters include benzoic acid, anisic acid, phenylacetic acid, cinnamic acid, salicylic acid, and anthranilic acid. Examples of alcohols that form the aliphatic and aromatic esters include straight-chain and branched aliphatic alcohols having 1 to 5 carbon atoms and the alcohols described above as examples of the fragrance components.

Examples of formic acid esters include linalyl formate, citronellyl formate, and geranyl formate.

Examples of acetic acid esters include ethyl acetate, isoamyl acetate (isopentyl acetate), benzyl acetate, hexyl acetate, phenyl acetate, p-cresyl acetate, cis-3-hexenyl acetate, linalyl acetate, citronellyl acetate, geranyl acetate, neryl acetate, terpinyl acetate, nopyl acetate, bornyl acetate, isobornyl acetate, acetyl eugenol, acetyl isoeugenol, o-tert-butylcyclohexyl acetate, p-tert-butylcyclohexyl acetate, tricyclodecenyl acetate, phenylethyl acetate, styralyl acetate, cinnamyl acetate, dimethylbenzylcarbinyl acetate, 3-pentyltetrahydropyran-4-yl acetate, prenyl acetate, geranyl acetate, and methylphenylcarbinyl acetate; and examples of phenylacetic acid esters include phenylethyl phenylacetate and p-cresyl phenylacetate.

Examples of propionic acid esters include ethyl propionate, citronellyl propionate, tricyclodecenyl propionate, benzyl propionate, styralyl propionate, and Helvetolide (trade name, manufactured by Firmenich; 2-[1-(3,3-dimethylcyclohexyl)ethoxy]-2-methylpropyl)propionate).

Examples of butyric acid esters include citronellyl butyrate, dimethylbenzylcarbinyl n-butyrate, isoamyl n-butyrate, and n-amyl n-butyrate; and examples of isobutyric acid esters include tricyclodecenyl isobutyrate and phenoxyethyl isobutyrate.

Examples of valeric acid esters include methyl valerate, ethyl valerate, butyl valerate, amyl valerate, benzyl valerate, and phenylethyl valerate; and examples of hexanoic acid esters include methyl hexanoate, ethyl hexanoate, allyl hexanoate, linalyl hexanoate, and citronellyl hexanoate.

Examples of heptanoic acid esters include methyl heptanoate and allyl heptanoate.

Examples of nonenoic acid esters include methyl 2-nonenoate, ethyl 2-nonenoate, and ethyl 3-nonenoate.

Examples of benzoic acid esters include methyl benzoate, benzyl benzoate, and 3,6-dimethyl benzoate.

Examples of cinnamic acid esters include methyl cinnamate and benzyl cinnamate.

Examples of salicylic acid esters include methyl salicylate, n-hexyl salicylate, cis-3-hexenyl salicylate, cyclohexyl salicylate, and benzyl salicylate (benzyl 2-hydroxybenzoate).

Examples of brassylic acid esters include ethylene brassylate.

Examples of tiglic acid esters include geranyl tiglate, 1-hexyl tiglate, and cis-3-hexenyl tiglate.

Examples of jasmonic acid esters include methyl jasmonate; and examples of dihydrojasmonic acid esters include MDJ (trade name, manufactured by Kao Corporation; methyl dihydrojasmonate, methyl(2-pentyl-3-oxocyclopentyl)acetate).

Examples of glycidic acid esters include methyl 2,4-dihydroxy-ethyl methylphenyl glycidate and 4-methylphenyl ethyl glycidate.

Examples of anthranilic acid esters include methyl anthranilate, ethyl anthranilate, and dimethyl anthranilate.

Examples of glycolic acid esters include allylcyclohexylglycolate.

Furthermore, examples of other esters include Ethyl Safranate (trade name, manufactured by Givaudan; ethyl dihydrocyclogeranate), Poirenate (trade name, manufactured by Kao Corporation; ethyl-2-cyclohexyl propionate), Fruitate (trade name, manufactured by Kao Corporation; ethyl tricyclo[5.2.1.0^{2.6}]decane-2-carboxylate), ethyl acetoacetate, Fructone (trade name, manufactured by IFF; ethyl 2-(2-methyl-1,3-dioxolan-2-yl)acetate), Manzanate (trade name, manufactured by Givaudan; ethyl 2-methylpentanoate), ethyl 2-methylbutyrate, allylcyclohexylpropionate, and allyl-2-pentyloxyglycolate.

Examples of the carbonates include Liffarome (trade name, manufactured by IFF; cis-3-hexenyl methyl carbonate), Jasmacyclat (trade name, manufactured by Kao Corporation; methyl-cyclooctyl carbonate), and Floramat (trade name, manufactured by Kao Corporation; ethyl-2-t-butylcyclohexyl carbonate).

Examples of the aldehydes include n-octanal, n-nonanal, n-decanal, n-dodecanal, 2-methylundecanal, 10-undecenal, citronellal, citral, hydroxycitronellal, Triplal (trade name, manufactured by IFF; 2,4-dimethyl-3-cyclohexene-1-carboxyaldehyde), Cyclovertal (trade name, manufactured by Kao Corporation; dimethyl-3-cyclohexenyl-1-carboxaldehyde), benzaldehyde, phenylacetaldehyde, phenylpropyl aldehyde, cinnamic aldehyde, dimethyltetrahydrobenzaldehyde, Bourgeonal (trade name, manufactured by Givaudan; 3-(4-tert-butylphenyl)propanal), Lyral (trade name, manufactured by IFF; hydroxy myrac aldehyde), Pollenal II (trade name, manufactured by Kao Corporation; 2-cyclohexyl propanal), Lilial (trade name, manufactured by Givaudan; p-tert-butyl-α-methylhydrocinnamic aldehyde), p-isopropyl-α-methylhydrocinnamic aldehyde, Floralozone (trade name, manufactured by IFF; 3-(o-(and p-)ethylphenyl)-2,2-dimethylpropionaldehyde), α-amyl cinnamic aldehyde, α-hexyl cinnamic aldehyde, Heliotropin (trade name, manufactured by Takasago International Corporation; 3,4-methylenedioxybenzaldehyde), Helional (trade name, manufactured by IFF; alpha-methyl-1,3-benzodioxole-5-propanal), Canthoxal (trade name, manufactured by IFF; 2-methyl-3-(para-methoxyphenyl)propanal), and Aldehyde C-6 (trade name, manufactured by Kao Corporation; n-hexanal).

Examples of the ketones include methylheptenone, dimethyloctenone, 3-octanone, hexylcyclopentanone, dihydrojasmone, Veloutone (trade name, manufactured by Firmenich; 2,2,5-trimethyl-5-pentylcyclopentanone), Nectaryl (trade name, manufactured by Givaudan; 2-(2-(4-methyl-3-cyclohexen-1-yl)propyl)cyclopentanone), ionone, β-ionone, methyl ionone, methyl ionone-G, γ-methyl ionone, damascone, α-damascone, β-damascone, δ-damascone, Isodamascone (trade name, manufactured by Symrise; 1-(2,4,4-trimethyl-2-cyclohexyl)-trans-2-butanone), damascenone, Dynascone (trade name, manufactured by Firmenich; 1-(5,5-dimethyl-1-cyclohexen-1-yl)-4-penten-1-one), irone, Cashmeran (trade name; manufactured by IFF; 1,2,3,5,6,7-hexahydro-1,1,2,3,3-pentamethyl-4H-inden-4-one), Iso E Super (trade name, manufactured by IFF; 1-(1,2,3,4,5,6,7,8-octahydro-2,3,8,8-tetramethyl-2-naphthalenyl)-ethan-1-one), Calone (trade name, manufactured by Firmenich; 7-methyl-3,4-dihydro-2H-benzodioxepin-3-one), carvone, menthone, acetyl cedrene, isolongifolanone, nootkatone, benzylacetone, raspberry ketone, benzophenone, Tonalide (trade name, manufactured by PFW; 6-acetyl-1,1,2,4,4,7-hexamethyltetrahydronaphthalene), β-methylnaphthyl ketone, ethyl maltol, camphor, muscone, Muscenone (trade name, manufactured by Firmenich; 3-methyl-5-cyclopentadecen-1-one), civetone, Glovanon (trade name, manufactured by Symrise; 8-cyclohexadecenone), methylnonyl ketone, cis-jasmone, and para-amylcyclohexanone (4-pentyl cyclohexanone). Of these, ionone, damascenone, Iso E Super, or γ-methyl ionone is preferable from the viewpoint of enhancing floral sweetness when blended with other fragrances.

Examples of the acetals include acetaldehyde ethylphenylpropyl acetal, citral diethyl acetal, phenylacetaldehyde glyceryl acetal, ethyl acetoacetate ethylene glycol acetal, Boisambrene Forte (trade name, manufactured by Kao Corporation; ethoxymethyl cyclododecyl ether), Troenan (trade name, manufactured by Kao Corporation; 5-methyl-5-propyl-2-(1-methylbutyl)-1,3-dioxane), Floropal (trade name, manufactured by Symrise; 2,4,6-trimethyl-4-phenyl-1,3-dioxane), and Magnolan (trade name, manufactured by Symrise; 2,4-dimethyl-4,4a,5,9b-tetrahydroindeno[1,2-d][1,3]dioxane).

Examples of the ethers include cedryl methyl ether, estragole, anethole, β-naphthyl methyl ether, β-naphthyl ethyl ether, limonene oxide, rose oxide, nerol oxide, 1,8-cineole, rosefuran, Ambroxan (trade name, manufactured by Kao Corporation; [3aR,-(3aα,5aβ,9aα,9bβ)]dodecahydro-3a,6,6,9a-tetramethylnaphtho[2,1-b]furan), Herbavert (trade name, manufactured by Kao Corporation; 3,3,5-trimethylcyclohexyl ethyl ether), Galaxolide (trade name, manufactured by IFF; hexamethylhexahydrocyclopentabenzopyran), phenylacetaldehyde dimethylacetal, and methyl isoeugenol.

Examples of the carboxylic acids include benzoic acid, phenylacetic acid, cinnamic acid, hydrocinnamic acid, butyric acid, and 2-hexenoic acid.

Examples of the lactones include ambrettolide, γ-decalactone, δ-decalactone, γ-valerolactone, γ-nonalactone, γ-undecalactone, 6-hexalactone, γ-jasmolactone, whisky lactone, coumarin, cyclopentadecanolide, cyclohexadecanolide, 11-oxahexadecanolide, and butylidenephthalide.

Examples of the nitriles include tridecene-2-nitrile, geranyl nitrile, citronellyl nitrile, and dodecanenitrile.

Examples of the amines include indole, skatole, 6-methylquinoline, 6-isopropylquinoline, and isobutylquinoline.

Examples of the Schiff bases include aurantiol and ligantraal.

It is also preferable that the fragrance composition of the present invention containing the compound represented by the formula (I) and the alcohol having 8 or more and 14 or less carbon atoms further contains natural essential oils or natural extracts.

Examples of the natural essential oils and the natural extracts include oils, concretes, absolutes, resinoids, oleoresins, tinctures, infusions, and balsams of natural raw materials, such as orange, lemon, lime, bergamot, vanilla, mandarin, peppermint, spearmint, lavender, galbanum, chamomile, rosemary, eucalyptus, sage, basil, rose, rockrose, geranium, jasmine, ylang-ylang, anise, clove, ginger, nutmeg, cardamom, cedar, Japanese cypress, vetiver, patchouli, hay, lemongrass, labdanum, grapefruit, elemi oils, and bergamot oils.

The total content of other components other than the compound represented by the formula (I) and the alcohol having 8 or more and 14 or less carbon atoms in the fragrance composition of the present invention can be selected as appropriate according to, for example, the type of fragrance blend and the type and intensity of intended aromatic odor. However, the total content of the other components in a composition containing the fragrance composition of the present invention is preferably 1 mass% or more, more preferably 5 mass% or more, still more preferably 10 mass% or more; and is preferably 99 mass% or less, more preferably 80 mass% or less, and still more preferably 70 mass% or less.

The fragrance composition of the present invention may be used in combination with an oil that serves as a base and itself is odorless. Such an oil allows fragrance components to be uniformly mixed together and to be easily added to a product, thereby allowing an odor with a moderate intensity to be more easily imparted to the product. Examples of the oil include polyhydric alcohols such as ethylene glycol, propylene glycol, butylene glycol, and dipropylene glycol; esters such as isopropyl myristate, dibutyl adipate, and diethyl sebacate; hydrocarbons such as liquid paraffin and squalane; and surfactants such as polyoxyethylene alkyl ethers and sorbitan fatty acid esters.

Of these, polyhydric alcohols and esters are preferable as the oil from the viewpoint of improving solubility of all the fragrance components, and dipropylene glycol and isopropyl myristate are more preferable. The content of such an oil in the fragrance composition is preferably 0.01 mass% or more, more preferably 1 mass% or more, and still more preferably 5 mass% or more; and is preferably 95 mass% or less, more preferably 90 mass% or less, and still more preferably 80 mass% or less.

The present invention further provides a cleaning composition containing the fragrance composition of the present invention, a cosmetic containing the fragrance composition of the present invention, and a fiber treating composition containing the fragrance composition of the present invention.

The cleaning composition of the present invention is preferably a body cleaning composition, a cleaning composition for clothing, or a cleaning composition for hard surfaces, more preferably a body cleaning composition or a cleaning composition for clothing, and still more preferably a cleaning composition for clothing.

The body cleaning composition is, for example, a skin cleaning composition, a hair cleaning composition, or a soap composition, and is preferably a skin cleaning composition.

The cleaning composition for hard surfaces is, for example, an all-purpose cleaner or a cleaning composition for tableware.

The fiber treating composition of the present invention is preferably a fabric softener composition.

The cosmetic of the present invention, other than cleaning compositions, is preferably a perfume, a milky lotion, a skin lotion, or a sunscreen, and more preferably a perfume.

The cleaning composition of the present invention preferably contains an anionic surfactant in addition to the fragrance composition of the present invention, and may further contain a nonionic surfactant, a pH adjuster, a viscosity modifier, a solvent, an oil, a preservative, water, etc.

The fiber treating composition of the present invention preferably contains a cationic surfactant in addition to the fragrance composition of the present invention, and may further contain a pH adjuster, a solvent, an oil, a preservative, water, etc.

The perfume of the present invention may contain a solvent, water, or the like, in addition to the fragrance composition of the present invention.

The compound represented by the formula (I) has a woody odor. This woody odor exhibits a minty and camphor-like characteristic with slight earthy and floral-muguet facets. This woody odor further possesses nectar-like sweetness. Furthermore, a composition consisting of the compound represented by the formula (I) and the alcohol having 8 or more and 14 or less carbon atoms can create a new impression by imparting naturalness to the alcohol having 8 or more and 14 or less carbon atoms.

Accordingly, the present invention provides a method of using the compound represented by the formula (I) of the present invention, or a composition composed of the compound represented by the formula (I) and the alcohol having 8 or more and 14 or less carbon atoms, as a fragrance-imparting component. Specifically, the present invention provides a method of using the same as a fragrance-imparting component in a fragrance composition, a cleaning composition, a cosmetic, or a fiber treating composition. The cleaning composition is preferably a body cleaning composition, a cleaning composition for clothing, or a cleaning composition for hard surfaces, more preferably a body cleaning composition or a cleaning composition for clothing, and still more preferably a cleaning composition for clothing. The body cleaning composition is, for example, a skin cleaning composition, a hair cleaning composition, or a soap composition, and is preferably a skin cleaning composition. The cleaning composition for hard surfaces is, for example, an all-purpose cleaner or a cleaning composition for tableware. The cosmetic is preferably a perfume. The fiber treating composition is preferably a fabric softener composition.

In the above-described method of use, the compound represented by the formula (I) of the present invention or the composition composed of the compound represented by the formula (I) and the alcohol having 8 or more and 14 or less carbon atoms is used in an amount of preferably 0.0001 mass% or more and more preferably 0.001 mass% or more, and preferably 30 mass% or less, more preferably 20 mass% or less, still more preferably 10 mass% or less, 1 mass% or less, and yet still more preferably 0.1 mass% or less, relative to the cleaning composition, the cosmetic, or the fabric softener composition. When used in such an amount, this fragrance composition can create a new impression by imparting a woody odor when is formulated as a fragrance material with other components.

In the above-described method of use, the fragrance composition of the present invention may be used in combination with an oil that itself is odorless. The oil is the same as that described above in connection with the fragrance composition. In addition, in the above-described method of use, a commonly used other fragrance component or a fragrance blend having a desired composition may also be contained as an additional fragrance. Such additional fragrances are the same as those described above in connection with the fragrance composition.

The present invention includes the following embodiments.
<1> A compound represented by a formula (I).

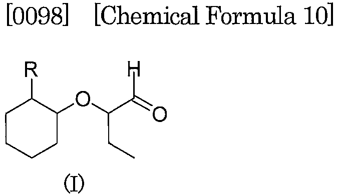

[In the formula (I),
R is an alkyl group having 1 or more and 5 or less carbon atoms.]

<2> The perfuming agent according to <1>, wherein R is a t-butyl group.

<3> A fragrance composition containing the compound according to <1> or <2>.

<4> The fragrance composition according to any one of <1> to <3>, wherein a content of the compound of the formula (I) in the fragrance composition is 0.05 mass% or more and 30 mass% or less.

<5> The fragrance composition according to any one of <1> to <4>, wherein a content of the compound of the formula (I) in the fragrance composition is 0.5 mass% or more and 20 mass% or less.

<6> The fragrance composition according to any one of <1> to <5>, wherein a content of the compound of the formula (I) in the fragrance composition is 1 mass% or more and 10 mass% or less.

<7> The fragrance composition according to any one of <3> to <6>, further containing an alcohol having 8 or more and 14 or less carbon atoms.

<8> The fragrance composition according to <7>, wherein the alcohol having 8 or more and 14 or less carbon atoms is an alcohol-based fragrance compound.

<9> The fragrance composition according to <7> or <8>, wherein the alcohol having 8 or more and 14 or less carbon atoms is a monohydric alcohol.

<10> The fragrance composition according to any one of <7> to <9>, wherein the alcohol having 8 or more and 14 or less carbon atoms is selected from the group consisting of Bacdanol (2-ethyl-4-(2,2,3-trimethyl-3-cyclopenten-1-yl)-2-buten-1-ol), cinnamic alcohol, citronellol, geraniol, phenethyl alcohol, dihydromyrcenol, Sandalmysore Core (2-methyl-4-(2,2,3-trimethyl-3-cyclopenten-1-yl)-2-buten-1-ol), Amber Core (1-(2-t-butylcyclohexyloxy)-2-butanol), linalool, myrcenol, nerol, tetrahydrolinalool, tetrahydrogeraniol, hydroxycitronellol, terpineol, isopulegol, nopol, thymol, eugenol, and 3-methyl-5-phenylpentanol.

<11> The fragrance composition according to any one of <7> to <10>, wherein the alcohol having 8 or more and 14 or less carbon atoms is one or more selected from the group consisting of Bacdanol, Sandalmysore Core, and Amber Core.

<12> The fragrance composition according to <7>, wherein the alcohol having 8 or more and 14 or less carbon atoms includes a compound represented by a formula (II).

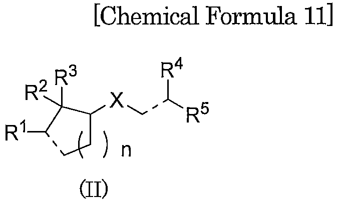

[In the formula (II),
R¹ and R² are each independently a hydrogen atom or a methyl group,
R³ is a methyl group, ethyl group, n-propyl group, isopropyl group, or t-butyl group,
R⁴ is a methyl group or ethyl group,
R⁵ is a hydroxyl group or hydroxymethyl group,
X is -CH₂- or -O-,
n is 1 or 2, and
each dashed line independently represents a single or double bond.]

<13> The fragrance composition according to any one of <7> to <10>, wherein the alcohol having 8 or more and 14 or less carbon atoms is one or more selected from the group consisting of 2-ethyl-4-(2,2,3-trimethyl-3-cyclopenten-1-yl)-2-buten-1-ol, 2-methyl-4-(2,2, 3-trimethyl-3-cyclopenten-1-yl)-2-buten-1-ol, and 1-(2-t-butylcyclohexyloxy)-2-butanol.

<14> The fragrance composition according to any one of <7> to <13>, wherein a mass ratio of the compound represented by the formula (I) to the alcohol having 8 or more and 14 or less carbon atoms [compound represented by formula (I)/alcohol having 8 or more and 14 or less carbon atoms] is 0.001 or more and 20 or less.

<15> The fragrance composition according to any one of <7> to <14>, wherein a mass ratio of the compound represented by the formula (I) to the alcohol having 8 or more and 14 or less carbon atoms [compound represented by formula (I)/alcohol having 8 or more and 14 or less carbon atoms] is 0.01 or more and 10 or less.

<16> The fragrance composition according to any one of <7> to <15>, wherein a mass ratio of the compound represented by the formula (I) to the alcohol having 8 or more and 14 or less carbon atoms [compound represented by formula (I)/alcohol having 8 or more and 14 or less carbon atoms] is 0.05 or more and 5 or less.

<17> The fragrance composition according to any one of <7> to <16>, wherein a mass ratio of the compound represented by the formula (I) to the alcohol having 8 or more and 14 or less carbon atoms [compound represented by formula (I)/alcohol having 8 or more and 14 or less carbon atoms] is 0.07 or more and 1 or less.

<18> The fragrance composition according to any one of <7> to <17>, further containing at least one component that is other than the compound represented by the formula (I) and the alcohol having 8 or more and 14 or less carbon atoms and is preferably selected from alcohols, phenols, esters, carbonates, aldehydes, ketones, acetals, ethers, lactones, nitriles, and amines.

<19> The fragrance composition according to any one of <7> to <18>, wherein a total content of other components other than the compound represented by the formula (I) and the alcohol having 8 or more and 14 or less carbon atoms, preferably a total content of alcohols, phenols, esters, carbonates, aldehydes, ketones, acetals, ethers, lactones, nitriles, and amines, is 1 mass% or more and 99 mass% or less.

<20> The fragrance composition according to any one of <7> to <19>, wherein a total content of other components other than the compound represented by the formula (I) and the alcohol having 8 or more and 14 or less carbon atoms, preferably a total content of alcohols, phenols, esters, carbonates, aldehydes, ketones, acetals, ethers, lactones, nitriles, and amines, is 5 mass% or more and 80 mass% or less.

<21> The fragrance composition according to any one of <7> to <20>, wherein a total content of other components other than the compound represented by the formula (I) and the alcohol having 8 or more and 14 or less carbon atoms, preferably a total content of alcohols, phenols, esters, a carbonate, an aldehyde, ketones, acetals, ethers, lactones, nitriles, and amines, is 10 mass% or more and 70 mass% or less.

<22> The fragrance composition according to any one of <7> to <17>, further containing a component that is other than the compound represented by the formula (I) and the alcohol having 8 or more and 14 or less carbon atoms and is preferably a natural essential oil or a natural extract.

<23> A cosmetic containing the fragrance composition according to any one of <3> to <22>.

<24> A cleaning composition containing the fragrance composition according to any one of <3> to <22>.

<25> A fiber treating agent composition containing the fragrance composition according to any one of <3> to <22>.

<26> A method of using the compound according to <1> or <2> as a fragrance-imparting component.

<27> A method of using a composition composed of the compound according to <1> or <2> and an alcohol having 8 or more and 14 or less carbon atoms as a fragrance-imparting component.

<28> A method for producing the compound according to <1>, the method including oxidizing a compound of a formula (III) to thereby obtain a compound of a formula (I).

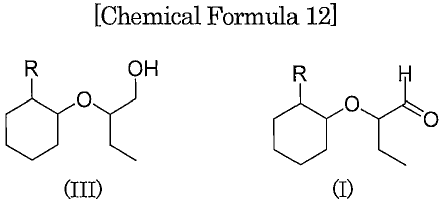

[In the formulae (I) and (III),
R is an alkyl group having 1 or more and 5 or less carbon atoms.]

In the following examples, "%" is "mass%", unless otherwise specified. The mass of a catalyst refers to the mass of the catalyst in a dry state.

### <Apparatus and Analytical Conditions for Gas Chromatography>

GC apparatus: 7890B, manufactured by Agilent Technologies, Inc., with hydrogen flame ionization detector

Column: For analysis of the yield, DB-1 (capillary column, 100% dimethylpolysiloxane, inner diameter: 0.25 mm, length: 30 m, film thickness: 0.25 µm, manufactured by Agilent Technologies, Inc.) was used.

For analysis of cis and trans isomers, DB-WAX (capillary column, polyethylene glycol, inner diameter: 0.25 mm, length: 30 m, film thickness: 0.25 µm, manufactured by Agilent Technologies, Inc.) was used.

### Carrier gas: He, 1.5 mL/min

Injection conditions: 300°C, split ratio: 100/1
Injection Amount: 1 µL
Detection conditions: FID System, 300°C
Column temperature conditions: Starting at 80°C, the temperature was raised to 300°C at a rate of 10°C/min, and then maintained at 300°C for 10 minutes.

### [Production Example 1]

### Production of 1-(2-t-butylphenyloxy)-2-butanol

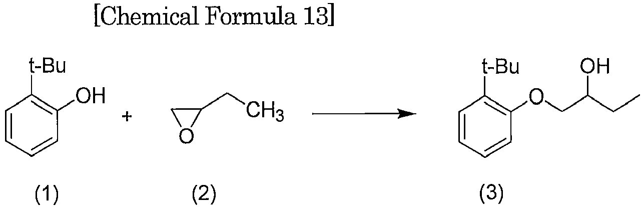

2-t-Butylphenol (1) (manufactured by FUJIFILM Wako Pure Chemical Corporation, 350 g) and 35 g of 48% sodium hydroxide aqueous solution (manufactured by Kanto Chemical Co., Inc.) were added to a 1-liter round-bottom flask provided with a Dimroth condenser and a dropping funnel in a nitrogen stream, and the resulting mixture was heated to 80°C. 1,2-Butylene oxide (2) (manufactured by Tokyo Chemical Industry Co., Ltd., 176 g) was added dropwise thereto over about 2 hours, followed by stirring at 80°C for 5 hours. After cooling the reaction mixture, an organic layer was separated from the underlying sodium hydroxide aqueous solution and then distilled, whereby 1-(2-t-butylphenyloxy)-2-butanol (3) was obtained in a yield of 96%.

### [Production Example 2]

### Production of mixture of 1-(2-t-butylcyclohexyloxy)-2-butanol (4) and 2-(2-t-butylcyclohexyloxy)-1-butanol (5)

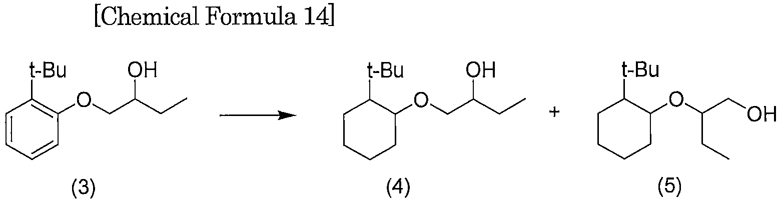

To a 500 ml autoclave, 1-(2-t-butylphenyloxy)-2-butanol (3) (250 g) obtained in Production Example 1, an activated carbon-supported palladium catalyst (manufactured by N.E. Chemcat Corporation, trade name: S-type, 50% water-containing product, palladium loading: 2%, 4.75 g), and an activated carbon-supported ruthenium catalyst (manufactured by N.E. Chemcat Corporation, 50% water-containing product, ruthenium loading: 5%, 0.25 g) were added, and the resulting mixture was reacted for 6 hours at 190°C and at a hydrogen pressure of 2.0 MPa.

After completion of the reaction, the catalysts were filtered off and the filtrate was distilled, whereby a mixture of 1-(2-t-butylcyclohexyloxy)-2-butanol (4) and 2-(2-t-butylcyclohexyloxy)-1-butanol (5) was obtained in a yield of 25%. Analysis of the product by gas chromatography showed that the mass ratio between 1-(2-t-butylcyclohexyloxy)-2-butanol (4) and 2-(2-t-butylcyclohexyloxy)-1-butanol (5) was (4):(5) = 75:25.

### [Example 1]

### Production of 2-(2-t-butylcyclohexyloxy)-butanal (6)

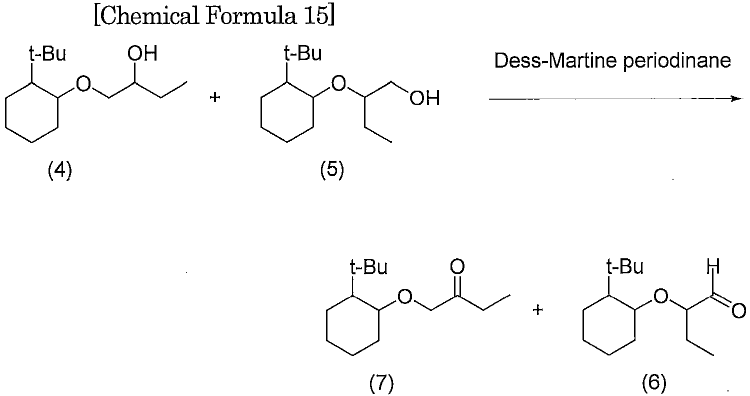

158 g of the mixture of 1-(2-t-butylcyclohexyloxy)-2-butanol (4) and 2-(2-t-butylcyclohexyloxy)-1-butanol (5), obtained in Production Example 2, was fractionally distilled using a 20-stage fractionating column at a reflux ratio of 20, whereby a mixture of 1-(2-t-butylcyclohexyloxy)-2-butanol (4) and 2-(2-t-butylcyclohexyloxy)-1-butanol (5) having a mass ratio of compound (4) to compound (5) of 31:69 was obtained in a yield of 50%.

The thus obtained mixture of 1-(2-t-butylcyclohexyloxy)-2-butanol (4) and 2-(2-(t-butylcyclohexyloxy)-1-butanol (5) (the mass ratio of compound (4) to compound (5) was 31:69) (1 g) was dissolved in dichloromethane (43.8 mL), and Dess-Martine periodinane (2.2 g) was added at 0°C. After 10 minutes, the cooling bath was removed, and the reaction mixture was warmed to room temperature and stirred for 30 minutes to carry out the reaction.

As a post-treatment, a saturated aqueous sodium thiosulfate solution was added to the reaction mixture to quench the reaction. The reaction mixture was extracted with diethyl ether. The organic phase was dried over sodium sulfate, the sodium sulfate was filtered off, and the solvent was then distilled off from the resulting filtrate to obtain a mixture of 2-(2-t-butylcyclohexyloxy)butanal (6) and 1-(2-t-butylcyclohexyloxy)butan-2-one (7).

An aqueous sodium bisulfite solution (100 mL) was added to the obtained mixture of 2-(2-t-butylcyclohexyloxy)butanal (6) and 1-(2-t-butylcyclohexyloxy)butan-2-one (7), and the resulting aqueous solution was stirred at room temperature (25°C) for 30 minutes. The reaction mixture was extracted with hexane (50 mL), and a 1 mol/L sodium hydroxide solution was then slowly added dropwise to the separated aqueous phase until the pH reached 10. The aqueous phase was stirred at room temperature (25°C) for 12 hours and then extracted with diethyl ether (100 mL). The organic phase was dried over sodium sulfate, the sodium sulfate was filtered off, and the solvent was then distilled off from the organic phase under reduced pressure to obtain a crude product. The resulting crude product was purified by silica gel column chromatography to obtain 2-(2-t-butylcyclohexyloxy)butanal (6) in a yield of 77%.

2-(2-t-Butylcyclohexyloxy)butanal (6) (diastereomer mixture) ¹H-NMR (400 MHz, CDCl₃):δ = 9.75 (s, 0.5H), 9.68 (s, 0.5H), 3.96 (m, 1H), 3.60 (m, 1H), 3.25 (m, 1H), 2.06 to 1.56 (m, 9H), 1.56 to 1.19 (m, 4H), 1.03 (s, 9H) ¹³C-NMR (100 MHz, CDCl₃):δ = 205.73, 204.82, 84.34, 82.50, 81.50, 79.74, 52.02, 51.51, 33.27, 31.10, 29.35, 29.28, 28.66, 28.56, 28.47, 26.56, 26.48, 25.82, 25.79, 24.44, 24.31, 24.22, 24.11, 9.22 IR (neat): 2931, 2862, 1731, 1078, 1013 cm⁻¹

### [Sensory Evaluation]

The odor of 2-(2-t-butylcyclohexyloxy)butanal (6) obtained in Example 1 and its effect in a fragrance blend were evaluated by three expert panelists. The evaluation was made using a fragrance test paper (Daimonji Paper Co., Ltd., 150 mm × 7 mm). 2-(2-t-Butylcyclohexyloxy)butanal (6) of Example 1 was applied to the tip of the fragrance test paper, and the evaluation was performed indoors. Sensory evaluation was made in terms of odor characteristics. In the sensory evaluation, the three expert panelists also described how they had felt about the quality and characteristics of the overall odor.

### [Evaluation of Example 1]

### [Odor Evaluation of 2-(2-t-Butylcyclohexyloxy)butanal (6)]

2-(2-t-Butylcyclohexyloxy)butanal (6) of Example 1 was confirmed to have a woody odor that exhibits a minty and camphor-like character with slight earthy and floral-muguet facets and possesses nectar-like sweetness.

### [Example 2: Blending Example 1]

A composition (Example 2) was prepared by adding 50 parts by mass of 2-(2-t-butylcyclohexyloxy)butanal (6), obtained in Example 1, to 950 parts by mass of a fragrance blend having a floral-rose note, the ingredients of which are shown in Table 1. A composition (Comparative Example 1) was prepared by adding 50 parts by mass of propylene glycol to 950 parts by mass of the same fragrance blend. Sensory evaluation was then carried out.

Note that, in Table 1, Bacdanol, cinnamic alcohol, citronellol, geraniol, and phenethyl alcohol are alcohols having 8 or more and 14 or less carbon atoms. In the composition of Example 2, the total amount of these alcohols having 8 or more and 14 or less carbon atoms was 41.5 mass%, and the amount of the compound of the formula (I) (2-(2-t-butylcyclohexyloxy)butanal (6)) was 5 mass%. The mass ratio of the compound represented by the formula (I) to the alcohol having 8 or more and 14 or less carbon atoms [compound represented by formula (I)/alcohol having 8 or more and 14 or less carbon atoms] was 0.12.

**[Table 1]**

| <Ingredients of Floral-Rose Fragrance Blend> | |
|---|---|
| Bacdanol (manufactured by IFF)^{*1)} | 10 parts by mass |
| Benzyl 2-hydroxybenzoate | 50 parts by mass |
| Cinnamic alcohol* | 5 parts by mass |
| Citronellol* | 100 parts by mass |
| Cyclopentadecanolide | 50 parts by mass |
| Damascenone | 0.5 parts by mass |
| Florosa (manufactured by Givaudan)²⁾ | 50 parts by mass |
| Geraniol* | 100 parts by mass |
| Geranyl acetate | 15 parts by mass |
| Heliotropin (manufactured by Takasago International Corporation)³⁾ | 10 parts by mass |
| Iso E Super (manufactured by IFF)⁴⁾ | 50 parts by mass |
| MDJ (manufactured by Kao Corporation)⁵⁾ | 100 parts by mass |
| Methyl isoeugenol | 5 parts by mass |
| Phenethyl alcohol* | 200 parts by mass |
| Dipropylene glycol | 204.5 parts by mass |
| Total | 950 parts by mass |

In Table 1, * indicates an alcohol having 8 or more and 14 or less carbon atoms.
1) Bacdanol: trade name, manufactured by IFF; 2-ethyl-4-(2,2,3-trimethyl-3-cyclopenten-1-yl)
2) Florosa: trade name, manufactured by Givaudan; 4-methyl-2-(2-methylpropyl)tetrahydro-2H-4-pyranol
3) Heliotropin: trade name, manufactured by Takasago International Corporation; 3,4-methylenedioxybenzaldehyde
4) Iso E Super: trade name, manufactured by IFF; 1-(1,2,3,4,5,6,7,8-octahydro-2,3,8,8-tetramethyl-2-naphthalenyl)-ethan-1-one
5) MDJ: trade name, manufactured by Kao Corporation; methyl-(2-pentyl-3-oxocyclopentyl)acetate

**[Table 2]**

| <Ingredients of Blending Examples> | | | | |
|---|---|---|---|---|
| | Floral-rose fragrance blend in Table 1 | Compound (6)*¹ of Example 1 | Dipropylene glycol | Total |
| Ex. 2 | 950 parts by mass | 50 parts by mass | - | 1000 parts by mass |
| Com. Ex. 1 | 950 parts by mass | - | 50 parts by mass | 1000 parts by mass |

| | | | | |
|---|---|---|---|---|
| *1: 2-(2-t-butylcyclohexyloxy)butanal (6) | | | | |

As a result of the sensory evaluation conducted by the three expert panelists, it was found that the fragrance composition of Example 2, as compared with the fragrance composition of Comparative Example 1, harmonized with the overall odor of the fragrance blend, and enhanced its naturalness, thereby rendering the odor of the fragrance blend closer to that of a natural fragrance.

### [Example 3: Blending Example 2]

A composition (Example 3) was prepared by adding 80 parts by mass of 2-(2-t-butylcyclohexyloxy)butanal (6), obtained in Example 1, to 920 parts by mass of a fragrance blend having a floral-oriental note, the ingredients of which are shown in Table 3 below. A composition (Comparative Example 2) was prepared by adding 80 parts by mass of dipropylene glycol to 920 parts by mass of the same fragrance blend. Sensory evaluation was then carried out.

Note that, in Table 3, dihydromyrcenol, Sandalmysore Core, and Amber Core are alcohols having 8 or more and 14 or less carbon atoms. In the composition of Example 3, the total amount of these alcohols having 8 or more and 14 or less carbon atoms was 12.9 mass%, and the amount of the compound of the formula (I) (2-(2-t-butylcyclohexyloxy)butanal (6)) was 5 mass%. The mass ratio of the compound represented by the formula (I) to the alcohol having 8 or more and 14 or less carbon atoms [compound represented by formula (I)/alcohol having 8 or more and 14 or less carbon atoms] was 0.38.

**[Table 3]**

| <Ingredients of Floral-Oriental Fragrance Blend> | |
|---|---|
| Bergamot oil | 80 parts by mass |
| Dihydromyrcenol* | 25 parts by mass |
| Allyl-2-pentyloxyglycolate | 5 parts by mass |
| Methylphenylcarbinyl acetate | 10 parts by mass |
| Ylang base | 50 parts by mass |
| Rose base | 50 parts by mass |
| Jasmine base | 100 parts by mass |
| MDJ (manufactured by Kao Corporation)⁵⁾ | 130 parts by mass |
| Methyl ionone gamma | 150 parts by mass |
| Sandalmysore Core (manufactured by Kao Corporation)*⁶⁾ | 50 parts by mass |
| Tonalide (manufactured by PFW Aroma Chemicals)⁷⁾ | 100 parts by mass |
| Benzyl salicylate | 50 parts by mass |
| Coumarin | 50 parts by mass |
| Vanillin | 20 parts by mass |
| Amber base (Amber Core)* | 50 parts by mass |
| Total | 920 parts by mass |

In Table 3, * indicates an alcohol having 8 or more and 14 or less carbon atoms.
5) MDJ: trade name, manufactured by Kao Corporation; methyl-(2-pentyl-3-oxocyclopentyl)acetate
6) Sandalmysore Core: trade name, manufactured by Kao Corporation; 2-methyl-4-(2,2,3-trimethyl-3-cyclopenten-1-yl)-2-buten-1-ol
7) Tonalide: trade name, manufactured by PFW Aroma Chemicals; 1-(3,5,5,6,8,8-hexamethyl-5,6,7,8-tetrahydronaphthalen-2-yl)ethan-1-one

**[Table 4]**

| <Ingredients of Blending Examples> | | | | |
|---|---|---|---|---|
| | Floral-oriental fragrance blend in Table 3 | Compound (6)*¹ of Example 1 | Dipropylene glycol | Total |
| Ex. 3 | 920 parts by mass | 80 parts by mass | - | 1000 parts by mass |
| Com. Ex. 2 | 920 parts by mass | - | 80 parts by mass | 1000 parts by mass |

| | | | | |
|---|---|---|---|---|
| *1: 2-(2-t-butylcyclohexyloxy)butanal (6) | | | | |

As a result of the sensory evaluation conducted by the three expert panelists, it was found that the fragrance composition of Example 3, as compared with the fragrance composition of Comparative Example 2, harmonized with the overall odor of the fragrance blend, and enhanced the sweetness of the odor of the floral-oriental fragrance blend.

The compound represented by the formula (I) and the fragrance composition containing the compound represented by the formula (I), each being of the present invention, have a woody odor. This woody odor exhibits a minty and camphor-like character with slight earthy and floral-muguet facets. This woody odor further possesses nectar-like sweetness.

## Claims

1. A compound represented by a formula (I): 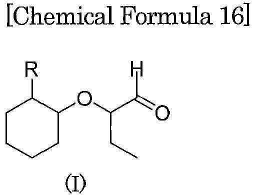 where
R is an alkyl group having 1 or more and 5 or less carbon atoms.

2. The compound according to claim 1, wherein R is a t-butyl group.

3. A fragrance composition comprising the compound according to claim 1 or 2.

4. The fragrance composition according to claim 3, further comprising an alcohol having 8 or more and 14 or less carbon atoms.

5. The fragrance composition according to claim 4, wherein the alcohol having 8 or more and 14 or less carbon atoms includes a compound represented by a formula (II): 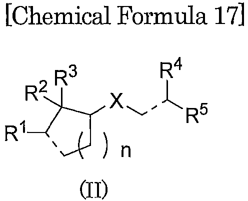 where
R¹ and R² are each independently a hydrogen atom or a methyl group,
R³ is a methyl group, ethyl group, n-propyl group, isopropyl group, or t-butyl group,
R⁴ is a methyl group or ethyl group,
R⁵ is a hydroxyl group or hydroxymethyl group,
X is -CH₂- or -O-,
n is 1 or 2, and
each dashed line independently represents a single or double bond.

6. The fragrance composition according to claim 4 or 5, wherein the alcohol having 8 or more and 14 or less carbon atoms is one or more selected from the group consisting of 2-ethyl-4-(2,2,3-trimethyl-3-cyclopenten-1-yl)-2-buten-1-ol, 2-methyl-4-(2,2,3-trimethyl-3-cyclopenten-1-yl)-2-buten-1-ol, and 1-(2-t-butylcyclohexyloxy)-2-butanol.

7. The fragrance composition according to any one of claims 4 to 6, wherein a mass ratio of the compound represented by the formula (I) to the alcohol having 8 or more and 14 or less carbon atoms [compound represented by formula (I)/alcohol having 8 or more and 14 or less carbon atoms] is 0.001 or more and 20 or less.

8. The fragrance composition according to any one of claims 4 to 7, further comprising at least one component that is other than the compound represented by the formula (I) and the alcohol having 8 or more and 14 or less carbon atoms and is selected from alcohols, phenols, esters, carbonates, aldehydes, ketones, acetals, ethers, lactones, nitriles, and amines.

9. A cosmetic comprising the fragrance composition according to any one of claims 3 to 8.

10. A cleaning composition comprising the fragrance composition according to any one of claims 3 to 8.

11. A fiber treating agent composition comprising the fragrance composition according to any one of claims 3 to 8.

12. A method of using the compound according to claim 1 or 2 as a fragrance-imparting component.

13. A method for producing the compound according to claim 1, the method comprising oxidizing a compound of a formula (III) to thereby obtain a compound of a formula (I): 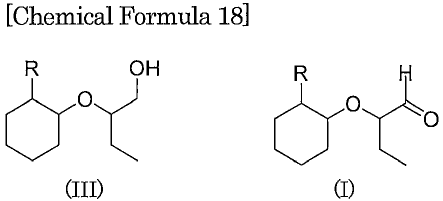 where
R is an alkyl group having 1 or more and 5 or less carbon atoms.
